# EUROPEAN PATENT SPECIFICATION

(11) **EP 3 682 845 B1**
(45) Date of publication and mention of the grant of the patent: **25.01.2023**
(21) Application number: 18871037.0
(22) Date of filing: 24.10.2018
(51) Int. Cl.: A61C 17/00, A46B 5/04, D04H 1/435, D04H 1/4382, A61C 19/06

(54) **CLEANING SLEEVE FOR CLEANING ORAL CAVITY**
REINIGUNGSHÜLSE ZUR REINIGUNG EINER MUNDHÖHLE
MANCHON DE NETTOYAGE POUR NETTOYER UNE CAVITÉ BUCCALE

(30) Priority: 26.10.2017 CN 201711017114
(43) Date of publication of application: 22.07.2020
(73) Proprietor: Hangzhou Jade Clove Science&Technology Co., Ltd., Hangzhou, Zhejiang 310052 (CN)
(72) Inventor: GONG, Ye, Hangzhou Zhejiang 310051 (CN)
(74) Representative: JD&P Patent Attorneys Joanna Dargiewicz & Partners
(86) International application number: PCT/CN2018/111524
(87) International publication number: WO 2019/080849

(56) References cited:
- CN-A- 1 316 211
- CN-A- 1 420 730
- CN-A- 107 693 143
- CN-U- 204 120 283
- CN-U- 205 556 987
- US-A- 3 902 509
- US-A- 5 107 562

## Description

### Technical field

The present invention relates to an oral cleaning tool, in particular to a cleaning sleeve for cleaning the oral cavity, wherein the cleaning sleeve can clean the coated tongue, gums, inner lips, upper jaws and lower jaws in the oral cavity.

### Background

Embarrassing situations may occur when people with halitosis communicate with others in public. The oral cavity is the beginning of the digestive tract and is also connected with the respiratory tract. One common complication caused by digestive system diseases and respiratory system diseases is halitosis. The degree of halitosis depends on different diseases such as disorder of digestion, gastroenteritis, diarrhea or constipation. Halitosis can also be observed from patients with bronchiectasis or pulmonary infection. In addition, patients who suffered from endocrine-related diseases such as diabetes may expel a scent of rotten apples from the oral cavity. With that in mind, halitosis may be a warning sign of disease. Consumption of spicy food, alcohol or coffee might also cause halitosis. The impact of halitosis might not only spoil one's appetite, but can also cause damage to one's mood and interpersonal relationships. Mouthwash may have a certain degree of ability to prevent halitosis, but can only yield short-term results. Moreover, alcoholic mouthwash may even intensify the symptoms of halitosis.

Research has shown that multiple factors involved in halitosis originated from oral bacteria. Bacteria may easily develop in the tongue base, hypoglottis, gums, inner lips, upper jaws and lower jaws as well as coated tongue. The bacteria in the tongue may produce germs, thus causing halitosis, or even stomatitis and periodontitis.

People usually brush their teeth every day. The benefits achieved by toothbrush and mouthwash comprise the cleaning of bacteria and the freshening of breath. However, the problem of halitosis is rarely realized by most people, e.g., little focus is given to the regular cleaning of the coated tongue or inner lips. Some people may try to use the toothbrush to brush the coated tongue, hypoglottis, upper jaws and lower jaws; but bristles of toothbrush in general are too rough for inner soft portions of the oral cavity. If the brushing force by hand is too strong, the inner soft portions could be easily damaged and symptoms of nausea may occur to cause uncomfortable feeling.

A plurality of oral cleaning finger cots for babies are created in the prior art, are typically made from plastic materials or silica gel and are not suitable for disposable use due to high costs. For example, CN103598722A discloses a baby toothbrush with a silica gel finger cot type structure that is equipped with bristles on one side and convex silica gel particles on the opposite. CN206062509U discloses a tongue cleaning brush comprising a cylindrical brush sleeve body made from soft silica gel. The brush sleeve body comprises a finger cavity that allows an adult index finger to fit inside, and also comprises an integrally formed brush sleeve body, a fixation portion at the end of the brush sleeve body, and a circular arc portion at the front edge of the brush sleeve body. The surface of the fixation portion is provided with a bite-resistant sleeve; and the surface of the brush sleeve body comprises bristles for brushing teeth and coated tongue massaging particles for massaging the coated tongue. The oral cleaning finger cots mentioned above are basically just toothbrushes with complicated coated tongue cleaning structures, have high production costs and will be difficult to sell to the general public for daily use.

CN206214218U discloses a finger cot toothbrush for babies, comprising a finger cot body made of medical gauze. The finger cot toothbrush can be used to clean the oral cavity after being applied over a finger and dipping into water. The finger cot in the prior art comprises a finger cot body that is applied over the finger while in use and can be made of medical gauze or cotton and nonwoven fabrics. An elastic edge fraping structure is arranged on the bottom edge of the finger cot to automatically strap the finger. A silica gel layer is arranged in the finger cot body to isolate and seal the product from the exterior. On the bottom of the finger cot, a finger loop made by an elastic tape is arranged on one side for the insertion of another finger that is adjacent to the one inserted into the finger cot body. A user may insert an index finger into the finger cot body with the help of the finger loop arranged on a middle finger to provide stability before use. While in use, the user may moisten the finger cot with water. The silica gel layer of the finger cot is provided to isolate and seal the product from water. The finger cot body allows easy cleaning of the coated tongue, gums and milk teeth of a baby after putting the finger into the mouth of the baby. The finger loop will prevent the finger cot body from slipping from the middle finger during cleaning. After cleaning is completed, the finger loop can also be used to hang for storage purpose.

According to the descriptions above, the finger cots in the prior art need to be manufactured and processed through sewing to form structural shapes. Therefore, the finger cots will be very difficult to be mechanically manufactured in a large scale and the cost will be very high as well. The finger cots are also required to be stored at a certain location for future use. Under home environment, it is difficult to sterilize the finger cots and great potential hazards exist. The finger cots also fail to achieve the goal of disposable and fast consumption. The raw materials used in the finger cots comprise non-degradable silica gel and elastomeric materials. The disposable use of the materials is detrimental to the environment. Due to the complicated structure, complicated manufacturing process, high cost and non-degradable property, the finger cots are unsuitable for daily safe use by the general public.

US 5 107 562 A discloses a cleaning sleeve for cleaning the oral cavity according to the preamble of claim 1.

US 2001/025644 A1 discloses a cleaning sleeve comprising a grip portion and a turn up portion, and an opening between the turn up portion and the grip portion.

### Summary

A technical problem to be solved by the present invention is to provide an oral cleaning finger cot that can reduce or avoid the problems mentioned above.

To solve the technical problem mentioned above, the present invention proposes a cleaning sleeve for cleaning the oral cavity, wherein the cleaning sleeve can be applied over a finger or a tool to clean the inside of the oral cavity. The cleaning sleeve has a flat-layered structure and comprises a sleeve portion formed by laminated sheet-like fabrics and a grip portion formed by a single layer of sheet-like fabrics. The sleeve portion comprises a working layer and a sleeve layer. A head is formed by the working layer and the sleeve layer at the top of the cleaning sleeve. The grip portion is integrally extended from the working layer.

Preferably, when packaged, the grip portion can be folded to cover and protect the surface of the working layer. While in use, the grip portion can be unfolded from the state of covering the working layer to fix the cleaning sleeve and prevent the cleaning sleeve from slipping from the finger or the tool.

Preferably, the same piece of fabric is folded to form the working layer and the sleeve layer. A seamless head is thereby formed between the working layer and the sleeve layer at the top of the cleaning sleeve. The working layer and the sleeve layer form a first edge and a second edge with bonded seams at both sides of the cleaning sleeve.

Preferably, the cleaning sleeve is formed by folding a piece of degradable nonwoven fabric and bonding the two sides via ultrasonic welding. The degradable nonwoven fabric is made by blending polylactic acid (PLA) fiber and viscose fiber.

Preferably, the mass ratio range of PLA fiber and viscose fiber in the degradable nonwoven fabric is (80-20): (20-80).

Preferably, the degradable nonwoven fabric has a weight per square meter of 40-150 grams and a mesh number of 10-30 and adopts a semi-cross or full-cross mesh laying method.

Preferably, the grip portion is equipped with an opening for facilitating fixation.

Preferably, the sleeve layer has an inserting edge that is opposite to the head of the cleaning sleeve. The inserting edge is equipped with an inserting structure for facilitating insertion.

Preferably, the head of the cleaning sleeve is equipped with arc angles on both corners. The arc angle on the operation corner of the cleaning sleeve is greater than the other one.

Preferably, both corners at the end of the grip portion form an end arc angle that is identical to the arc angle on the corresponding corner of the head of the cleaning sleeve.

The cleaning sleeve of the present invention can be formed by folding a piece of degradable nonwoven fabric and bonding the two sides via ultrasonic welding. Since additional processes such as sewing and connecting are not required, the oral cleaning finger cot is easy in mass manufacturing, low in cost and therefore suitable for disposable use. The general public can afford to use the cleaning sleeves on a daily basis with low prices. In addition, the thermal bonding effect and water locking property of the degradable nonwoven fabric achieve a perfect balance.

The grip portion of the cleaning sleeve of the present invention can be integrally extended from the working layer as the grip portion and the working layer are made of the same piece of fabric. Since additional processes such as sewing and connecting are not required, the cleaning sleeve is easy in mass manufacturing, low in cost and therefore suitable for disposable use. The general public can afford to use the cleaning sleeves on a daily basis with low prices.

In addition, the present invention is formed by folding a piece of degradable nonwoven fabric and bonding the two sides via ultrasonic welding. Since additional processes such as sewing and connecting are not required for the two, the cleaning sleeve is easy in mass manufacturing, low in cost and therefore suitable for disposable use. The general public can afford to use the cleaning sleeves on a daily basis with low prices.

In addition, the cleaning sleeve of the present invention can be formed via folding a piece of nonwoven fabric into a double-layer structure of the sleeve portion to eliminate the process of producing two separate layers. One nonwoven fabric layer is simply folded to form the two separate layers, thereby avoiding wasting materials as the process of die cutting while forming the head. With a simpler and safer processing method, the production capacity and efficiency can be greatly improved. In addition, such a manner of folding in the middle is adopted to eliminate the process of using ultrasonic bonding to form the head of the cleaning sleeve, thereby not only reducing processing losses, but also avoiding the tough corners caused by using an ultrasonic thermal bonding process at the top, and offering better user experience.

In addition, the grip portion of the cleaning sleeve of the present invention not only has a gripping and fixing function, but also offers a protective function when being folded to cover the working layer. The structure has two different functions to avoid the need of a separate piece of protective film or isolation paper and thus lower manufacturing costs and improve production efficiency while greatly improving the safeness and hygiene of products.

### Description of Drawings

The following drawings are only intended to illustrate and explain the present invention and do not limit the scope of the present invention.
Fig. 1 is a front schematic diagram of a cleaning sleeve for cleaning the oral cavity according to an example which is not part of the present invention;
Fig. 2 is a schematic diagram of the cleaning sleeve for cleaning the oral cavity as shown in Fig. 1 in a ready-to-use state;
Fig. 3 is a schematic diagram of the cleaning sleeve for cleaning the oral cavity as shown in Fig. 1 while in use;
Fig. 4 is a front schematic diagram of a cleaning sleeve for cleaning the oral cavity according to another example which is not part of the present invention;
Fig. 5 is a front structural schematic diagram of a cleaning sleeve for cleaning the oral cavity according to another example which is not part of the present invention;
Fig. 6 is a schematic diagram of the cleaning sleeve for cleaning the oral cavity as shown in Fig. 5 in the ready-to-use state;
Fig. 7 is a structural schematic diagram of a cleaning sleeve for cleaning the oral cavity according to an embodiment of the present invention;
Fig. 8 is a schematic diagram of the cleaning sleeve for cleaning the oral cavity as shown in Fig. 7 in the ready-to-use state;
Fig. 9 is a schematic diagram of the cleaning sleeve for cleaning the oral cavity as shown in Fig. 7 while in use;
Figs. 10-15 are schematic diagrams of various types of easy-to-insert structures of the cleaning sleeve for cleaning the oral cavity of the present invention;
Fig. 16 is a schematic diagram of the processing of a cleaning sleeve for cleaning the oral cavity according to another example which is not part of the present invention;
Fig. 17 is a schematic diagram of a cleaning sleeve for cleaning the oral cavity according to another embodiment of the present invention in a packaged state; and
Fig. 18 is a structural schematic diagram of another cleaning sleeve according to the present application.

### Detailed Description

For a better understanding of the technical features, objectives and effects of the present invention, the embodiments of the present invention will be described with reference to the accompanying drawings. The same reference number will denote the same element.

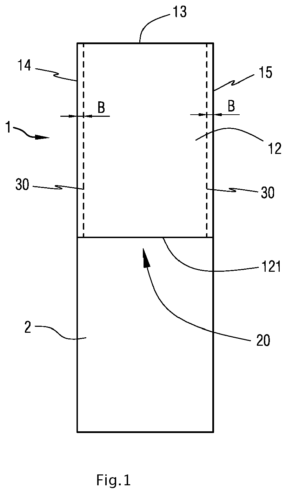

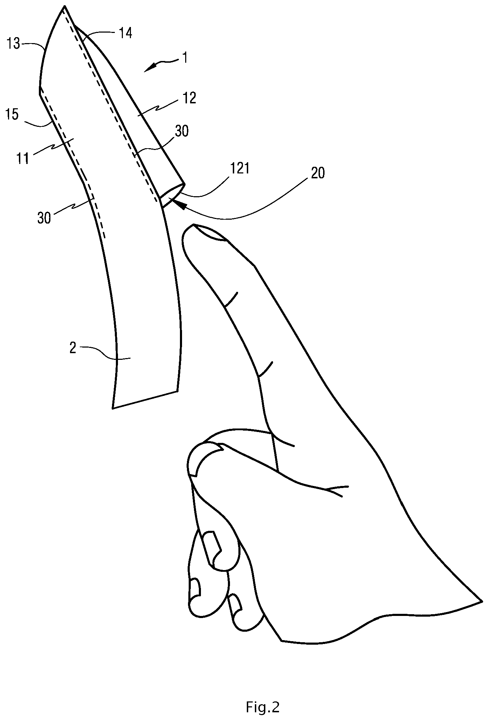

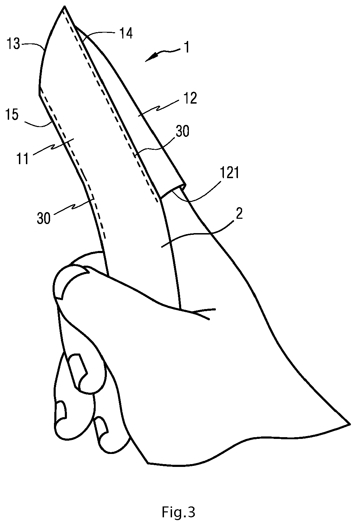

Based on the deficiencies of the prior art described above, the present invention provides a cleaning sleeve for cleaning the oral cavity, wherein the basic design concept and working principle of the cleaning sleeve are detailed in Figs. 1-3. Fig. 1 is a front schematic diagram of the cleaning sleeve for cleaning the oral cavity according to an example which is not part of the present invention. Fig. 2 is a schematic diagram of the cleaning sleeve for cleaning the oral cavity as shown in Fig. 1 in a ready-to-use state. Fig. 3 is a schematic diagram of the cleaning sleeve for cleaning the oral cavity as shown in Fig. 1 while in use.

Referring to Figs. 1-3, the cleaning sleeve for cleaning the oral cavity of an example which is not part of the present invention is suitable for being applied over a finger or a tool in order to clean the inside of the oral cavity. For example, as shown in Figs. 2 and 3, the cleaning sleeve of the present invention can be used by directly applying over a dominant finger (e.g., index finger) of a user or by means of a tool such as applying over a toothbrush head (not shown in the figures). Based on a basic structure, the cleaning sleeve of the present invention can be referred to as an oral cleaning finger cot (because it can be applied over the finger for use). However, it can also be applied over the surface of a tool (e.g., the toothbrush head). Therefore, the cleaning sleeve of the present invention contains the concept of the oral cleaning finger cot, but has a protection scope more extensive than that of the oral cleaning finger cot. In addition, the cleaning sleeve of the present invention is able to clean the coated tongue, gums, inner lips, upper jaws and lower jaws, tongue base and hypoglottis, and is not limited to clean only the teeth and coated tongue.

It can be seen from the figures that the general structure of the cleaning sleeve of the present invention is very visualized. The cleaning sleeve has a flat-layered structure and comprises a sleeve portion 1 formed by layered sheet-like fabrics and a grip portion 2 formed by singer-layer fabrics. The sleeve portion 1 comprises a working layer 11 and a sleeve layer 12. Since Fig. 1 is a plain view, the front side that faces an observer presents the sleeve layer 12 of the sleeve portion 1 and the backside presents the working layer 11 of the sleeve portion 1. The layered working layer 11 and sleeve layer 12 form a head 13 at the top of the cleaning sleeve. The two sides of the present invention form a first edge 14 and a second edge 15. The working layer 11 and the sleeve layer 12 are respectively connected with the head 13, the first edge 14 and the second edge 15 to form a sleeve-like structure with an inserting hole 20. For example, when the working layer 11 and the sleeve layer 12 are laminated together, the positions in the first edge 14 and the second edge 15 can be bonded via methods such as ultrasonic welding and form a bonded seam 30 (as shown in dotted line). In this way, the first edge 14 and the second edge 15 are finally jointed together. The sleeve layer 12 comprises an inserting edge 121 opposite to the head 13. The inserting edge 121 forms the edge of the inserting hole 20. The specific structure can be clearly seen with reference to Figs. 2 and 3.

From the structure of the cleaning sleeve of the present invention, it can be seen that the sleeve portion 1 is placed over a finger or the surface of a tool. The working layer 11 faces and wipes a position that needs to be cleaned. The sleeve layer 12 is adopted to ensure that the sleeve portion 1 is held outside the finger or the tool. The grip portion 2 can be fixed by hand to prevent the cleaning sleeve from slipping from the finger or the tool. For example, in Fig. 3, a user can hold the grip portion 2 inside the palm. If the sleeve portion 1 is applied over a toothbrush head, the user may hold the grip portion 2 and a toothbrush handle together in the palm or wrap the grip portion 2 around the toothbrush handle and then grip the grip portion 2 and the toothbrush handle together to achieve a more steady performance.

From the structure of the present invention, it can be seen that the structure of the cleaning sleeve of the present invention is a lot simpler than the structures in the prior art and may be directly processed by a piece of sheet-like fabric. For example, from Figs. 2 and 3, it can be seen that the grip portion 2 is integrally extended from the working layer 11, i.e., the grip portion 2 and the working layer 11 are made of the same piece of sheet-like fabric. Since additional processes such as sewing and connecting are not required for the two, the cleaning sleeve is easy in mass manufacturing, low in cost and therefore suitable for disposable use. The general public can afford to use the cleaning sleeves on a daily basis with low prices. Alternatively, the grip portion 2 can also be connected to the working layer 11 by a separate piece of fabric with the same shape as the grip portion 2. Namely, in the example, the sleeve portion 1 and the grip portion 2 can be separately manufactured and then the grip portion 2 can be connected with the working layer 11 of the sleeve portion 1 via ultrasonic bonding or other methods. From the structure of the two embodiments above, it can be seen that the grip portion 2 can be integrally extended from the working layer 11 and can also be a separate structure that can be connected with the working layer 11. In the present application, a preferred solution is that the grip portion 2 is defined to be integrally extended from the working layer 11, wherein the protection scope is different from the protection scope in such a situation that the grip portion 2 is not defined. Therefore, the protection scopes cannot be identical and repetitive.

In a preferred embodiment, the cleaning sleeve of the present invention can be made from a piece of degradable nonwoven fabric that is formed by materials such as polylactic acid (PLA) fiber. The cleaning sleeve can also be for sale after being infiltrated with liquid. The liquid infiltrated cleaning sleeve provides more frictional force and thus enhances the cleaning effect. The liquid for infiltrating the cleaning sleeve in the present invention is typically water or other liquids that have sterilization, deodorization and anti-inflammation effects. Certainly, the cleaning sleeve of the present invention can also be for sale in a dry state as consumers can moisten the cleaning sleeve with little water before performing the cleaning operation. The liquid infiltrated cleaning sleeve is relatively more convenient to use and the safeness can also be ensured since the consumers may not have access to clean water capable of infiltrating the cleaning sleeve in some cases. In addition, the cleaning sleeve of the present invention is made of degradable nonwoven fabrics, is able to be naturally degraded, and is thus friendly to our environment and suitable for disposable use by the general public.

The cleaning sleeve of the present invention can be manufactured into various shapes via die cutting of the sheet-like fabric. The sleeve portion 1 then can be formed via ultrasonic bonding of edges without manual sewing so that the cleaning sleeve can be manufactured in scale in the entire process. During manufacturing, interventions from the external environment are avoided, thus sterile production can be achieved and the product has high hygiene and safety. The product only uses little materials, therefore the cost can be very low and the product is very suitable for mass production and sales. The products with similar functions in the prior art fail to exhibit advantages such as easy production, low cost, safe and convenient operation and disposable use.

Furthermore, with regard to adoption of the ultrasonic bonding technology rather than sewing in the present invention, the former method can advance mass production, lower processing cost, yield higher productivity, have high product precision, easily achieve fully automation production and easily control the quality. In addition, the involvement of labor work is completely eliminated, thus the possibility of bringing bacteria is avoided and the hygiene and safety of the product can be greatly improved. The sewing method requires high labor cost, but has relatively low productivity and is very difficult to achieve fully automation production.

The bonded seam 30 indicated by the dotted line shown in Figs. 1-3 is not visible from the outside. The nonwoven fabric is heated and fused together via ultrasonic welding and the difference is not visible on the outer surface other than the fact that the fused bonded seam 30 is slightly rougher than other positions. Ultrasonic bonding is a well-known bonding technology that can process with the nonwoven fabrics suitable for ultrasonic bonding, wherein the materials must be thermoplastic materials. The thermoplastic materials such as polypropylene (PP), polyethylene (PE) and polyester are preferred. Certainly, due to increasingly strict environmental regulations, the use of thermoplastic materials such as polyester has led to the production of a large amount of non-degradable wastes and the inventor decided to focus on degradable materials. The inventor initially seeks for processing with nonwoven fabrics applicable to the ultrasonic bonding technology such as PLA fiber, which can meet requirements of ultrasonic bonding, is naturally degradable and contains great antibacterial ingredients. However, the PLA fiber has the above advantages, but has a certain disadvantage such as poor liquid absorption performance and failure to lock the moisture. Since the cleaning sleeve of the present invention needs to be used in wet conditions after the infiltration of liquid and requires excellent water locking performance, use requirements of the cleaning sleeve of the present invention cannot be satisfied when the cleaning sleeve is simply prepared from the PLA fiber.

Therefore, in a preferred embodiment, the cleaning sleeve of the present invention is formed by ultrasonically bonding the two sides of a folded piece of degradable nonwoven fabric, which is blended with PLA fiber and viscose fiber. The cleaning sleeve can also be for sale after being infiltrated with liquid.

Furthermore, the mass ratio of the PLA fiber to the viscose constituting the degradable nonwoven fabric is (80-20): (20-80).

As a result, to meet the requirements of the cleaning sleeve of the present invention such as use in a wet state, degradability, sterilization and ultrasonic bonding, the present invention provides a specific solution in which the nonwoven fabric for the cleaning sleeve of the present invention is prepared from two fibers with different components by a semi-cross mesh laying method rather than degradable fibers with a single component. As the proportion of the PLA fiber constituting the degradable nonwoven fabric of the present invention increases, the thermal bonding effects becomes better, but the water locking ability of the fabric deteriorates. Vice versa, as the proportion of the viscose fiber increases, the water locking ability of the fabric increases, but the thermal bonding ability deteriorates. After repeated verifications, the thermal bonding effect and the water locking ability of the degradable nonwoven fabric achieve a perfect balance at the ratio of the above embodiment.

In an embodiment, the PLA fiber used in the nonwoven fabric of the present invention may be PLA02 PLA fiber purchased from Zhejiang Hisun Biomaterials Co., Ltd.; and the viscose fiber may be 1.67dtex*38mm of high white and semi-dull viscose fiber purchased from Tangshan Sanyou Group Xingda Chemical Fiber Co., Ltd. Certainly, those skilled in the art can also purchase the same type of PLA fiber and viscose fiber from the market to prepare the nonwoven fabric for the cleaning sleeve of the present invention. Since PLA fiber is adopted in the nonwoven fabric of the present invention, the antibacterial and bacteriostatic effect of the product can be improved without adding preservatives, and the expiration date can also be prolonged, thereby eliminating concerns about preservatives of the consumers and improving product sales.

In another embodiment, the present invention further optimizes the preparation process of the nonwoven fabric to obtain a better user experience, improve the friction performance of the product and achieve a better effect of removing adhesive substances and bacteria. Specifically, different mesh forming methods enable the materials to have different physical properties. User experience can vary due to different fabric weights. Furthermore, the friction properties of the materials show imparities as mesh numbers of the nonwoven fabric varies. Therefore, the nonwoven fabric of the present invention preferably has a weight per square meter of 40-150 grams and a mesh number of 10-30, and adopts a semi-cross or full-cross mesh laying method.

Furthermore, the bonded seem 30 indicated by the dotted line in the figure is formed via ultrasonically heating and fusing the layered nonwoven fabric together. The difference is not visible from the outside other than the fact that the bonded seam 30 is slightly rougher than other positions. To avoid the discomfort of frictions in the oral cavity caused by the ultrasonically bonded rough corners, an un-bonded region preferably exists between the bonded seam 30 and the edge of the fabric. The width B of the un-bonded region is 0.1-4mm (as shown in Fig. 1). Namely, during the ultrasonic bonding, the bonded region is not at the edge of the fabric, but adjacent to the inside of the edge. Thus, a soft region is left outside the rough bonded seam 30 so that the cleaning sleeve is softer and more comfortable to use. Certainly, Fig. 1 is only a simple schematic diagram. The bonded seam 30 has a certain width between 0.5 mm and 5 mm to ensure the reliability of ultrasonic bonding.

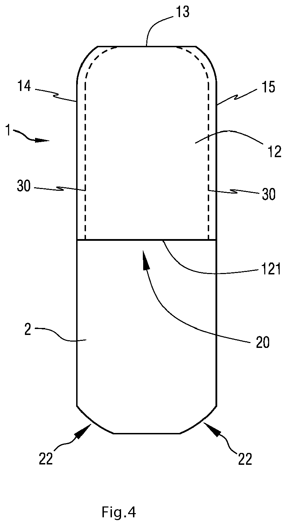

Furthermore, the present invention also makes some ergonomics improvement to provide a better user experience of the cleaning sleeve of the present invention. Fig. 4, which is not part of the present invention, is a front structural schematic diagram of the cleaning sleeve for cleaning the oral cavity according to another embodiment of the present invention. The figure shows that the working layer 11 and the sleeve layer 12 form the head 13 at the top of the cleaning sleeve. The head 13 has arc angles on both corners, thus reduces the irritation to the oral cavity caused by the initial right angle corners and provides better user experience. Moreover, the corners of the two sides at the end of the grip portion 2 can form the end arc angles 22 to provide a better visual curved corner.

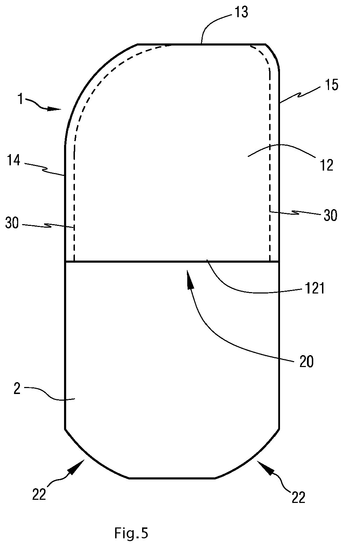

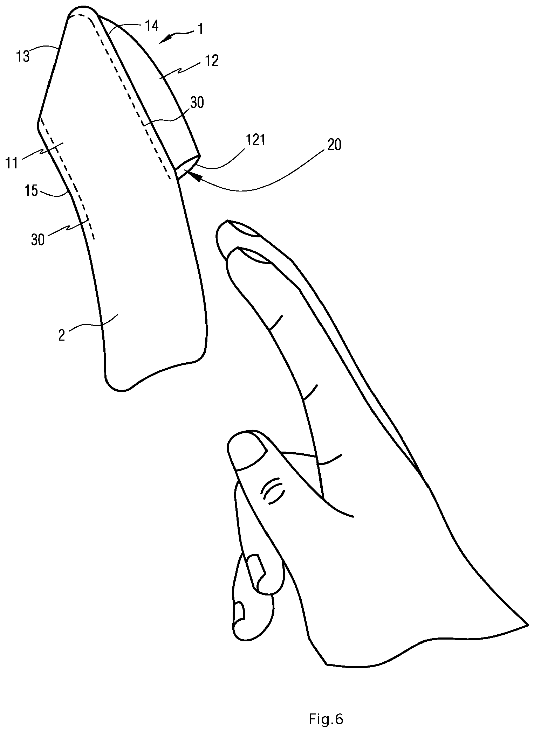

Fig. 5 is a front structural schematic diagram of the cleaning sleeve for cleaning the oral cavity according to another embodiment of the present invention. The figure shows that the cleaning sleeve of the present invention can also be applied with two fingers or a wider tool. Fig. 6 shows a schematic diagram of the cleaning sleeve for cleaning the oral cavity as shown Fig. 5 in a ready-to-use state. As shown in Figs. 5-6, since the width of the cleaning sleeve becomes wider, a wrinkled region will emerge when inserting two fingers with different lengths. Based on an ergonomic design concept, in order to avoid stimulating the oral cavity by a sharp corner formed by the wrinkled region, the head 13 of the cleaning sleeve according to the present embodiment has a bigger arc angle on the operation side than that on the other side. Therefore, the sleeve portion 1 of the cleaning sleeve is closer to the finger or the surface of the tool to enhance the user experience. For example, suppose a user inserts an index finger and a middle finger of the right hand into the cleaning sleeve shown in Figs. 5-6. The left side of the head 13 of the cleaning sleeve in the figure is the operation side as it is easier to apply pressure and more flexible while performing cleaning operation. Since the index finger is usually shorter than the middle finger, the arc angle of the head 13 of the cleaning sleeve at the operation side is designed to be greater to fit the index finger better. The wrinkled region on the side of the corresponding index finger will disappear. As the accumulation of wrinkled region might form a rough sharp corner to cause discomfort to the oral cavity, the elimination of it will facilitate use and provide better user experience. Certainly, those skilled in the art should understand that based on the demand of different consumer groups such the left-handers, the arc angle on the operation side of the head 13 of the cleaning sleeve of present invention may also be opposite to the direction shown in Figs. 5-6.

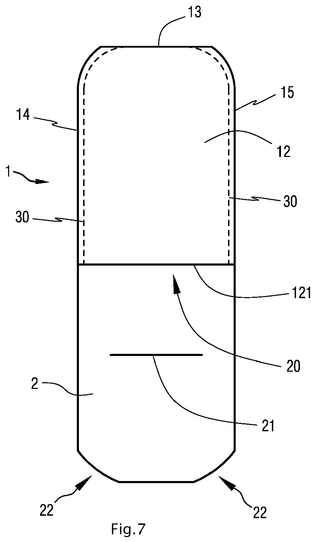

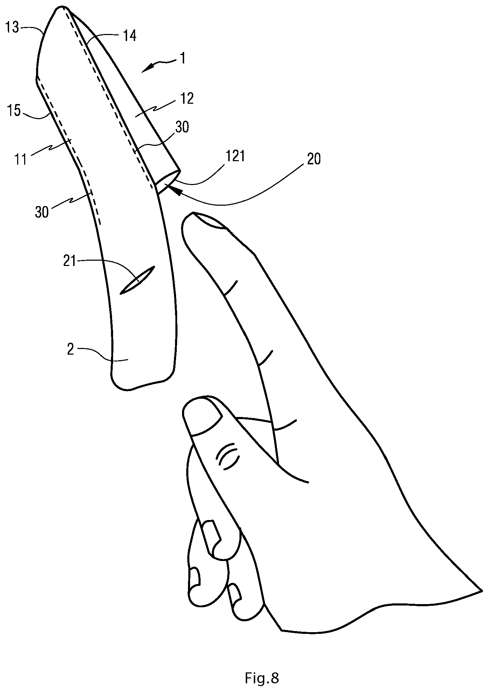

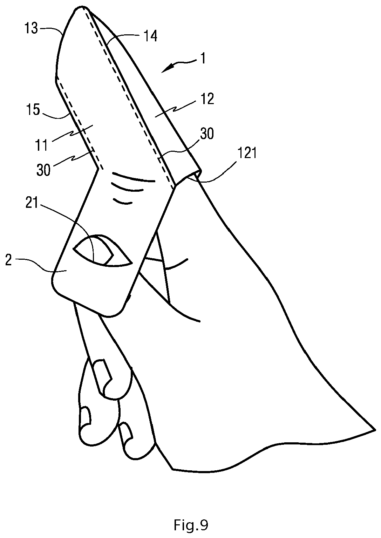

Furthermore, Fig. 7 shows a structural schematic diagram of the cleaning sleeve for cleaning the oral cavity according to an embodiment of the present invention. Fig. 8 is a schematic diagram of the cleaning sleeve for cleaning the oral cavity as shown in Fig. 7 in the ready-to-use state. Fig. 9 is a schematic diagram of the cleaning sleeve for cleaning the oral cavity as shown in Fig. 7 while in use. As shown in the figure, the grip portion 2 of the cleaning sleeve of the present embodiment has an opening 21 for inserting a finger to fix the cleaning sleeve in place. While in use, the grip portion 2 is fixed by inserting the thumb into the opening 21 to prevent the cleaning sleeve from slipping. The opening 21 can be arranged parallel to the edge of the head of the cleaning sleeve as shown in the figure. Alternatively, the opening 21 can be arranged perpendicular to the edge of the head of the cleaning sleeve (not shown in the figure). Based on user experience, the opening 21 can also be designed in holes of other shapes and the direction can be arbitrarily designed as long as a finger or other fixtures can be easily inserted to fix.

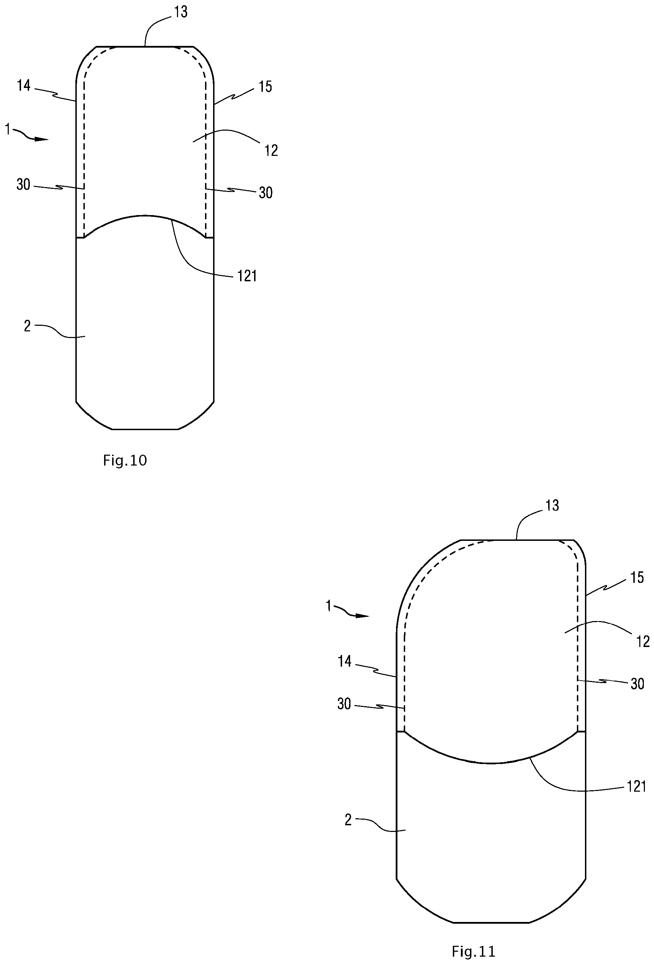

In addition, since the cleaning sleeve is infiltrated with liquid when in use, the working layer 11 and the sleeve layer 12 of sleeve portion 1 of the cleaning sleeve are easily attached together under the surface tension of the liquid and thus it is not easy to open the sleeve portion 1 to insert the finger or the tool into the sleeve portion 1 through the inserting hole 20. To solve the problem, the present invention provides a series of technical solutions to make an easier insertion for a finger or a tool as shown in Figs. 10-15 respectively. The figures sequentially represent schematic diagrams of various types of easy-to-insert structures of the cleaning sleeve of the present invention.

Particularly, in Figs. 10-15, the present example which is not part of the present invention provides various improvements of the sleeve layer 12 of the cleaning sleeve. The sleeve layer 12 has as inserting edge 121 opposite to the head 13. The inserting edge 121 forms the edge of the inserting hole 20 and provides an inserting structure for facilitating insertion.

Figs. 10-11 respectively show that the inserting structure is a curved edge of the inserting edge 121 facing or deviating from the head 13. The length of the curved edge is longer than the straight edge and is more easily bent. Therefore, the curved edge is easier to be opened than the straight edge and thus easier to be inserted.

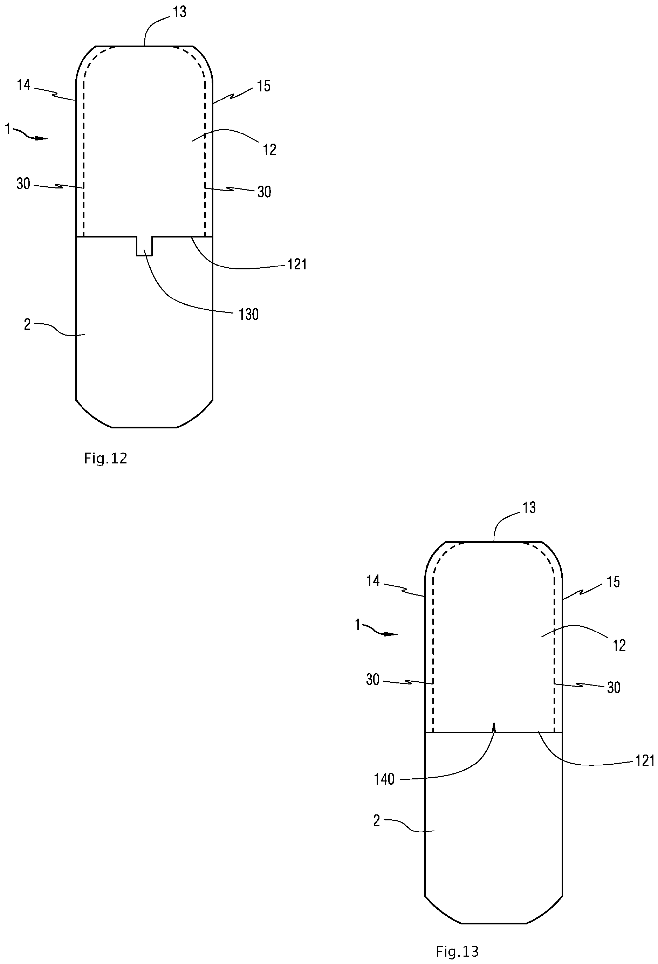

As shown in Fig. 12, the inserting structure is a pulling edge 130 extended from the inserting edge 121, and helps the sleeve portion 12 to be pulled and opened easily. The pulling edge 130 shown in Fig. 12 can be a relatively narrow pulling edge (as shown in Fig. 12) or a relatively wide edge (not shown in the figure) extending along the entire length of the inserting edge 121. The pulling edge 130 can be an integral extension of the sleeve layer 12 and can also be a pull-tab that is attached to the inserting edge 121. Alternatively, the pulling edge 130 can be further simplified into a thin line, which can be a thin line formed by overall extension of the sleeve layer 12 and can also be an easy-to-pull thin line attached to the inserting edge 121.

As shown in Fig. 13, the inserting structure is a notch 140 formed in the middle of the inserting edge 121. The notch 140 breaks the surface tension of the linear structure of the inserting edge 121, reduces the bonding force and allows a smoother insertion.

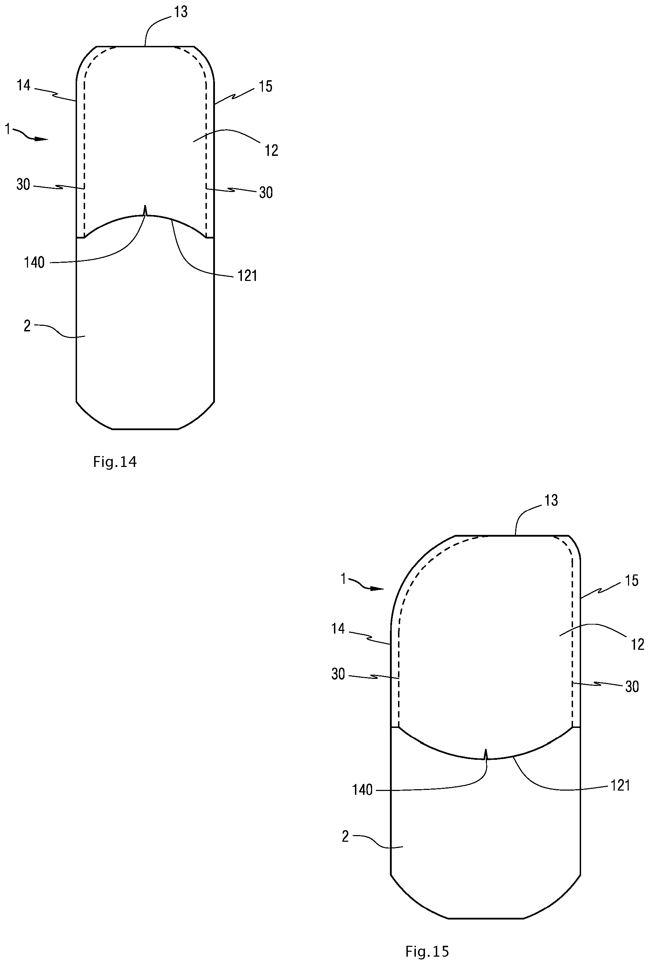

As shown in Figs. 14-15, the inserting structure is a curved edge that faces or deviates from the head 13; and a notch 140 is formed in the middle of the curved edge. Namely, the examples which are not part of the present invention shown in Figs. 14-15 respectively are improved structures in which the notch 140 of Fig. 13 is added on the basis of Figs. 10-11 to achieve a smoother insertion.

Furthermore, the present invention also provides a further optimized modification of the cleaning sleeve based on the various embodiments mentioned above, which can further improve the comfort and usability of the product and reduce the cost of the product.

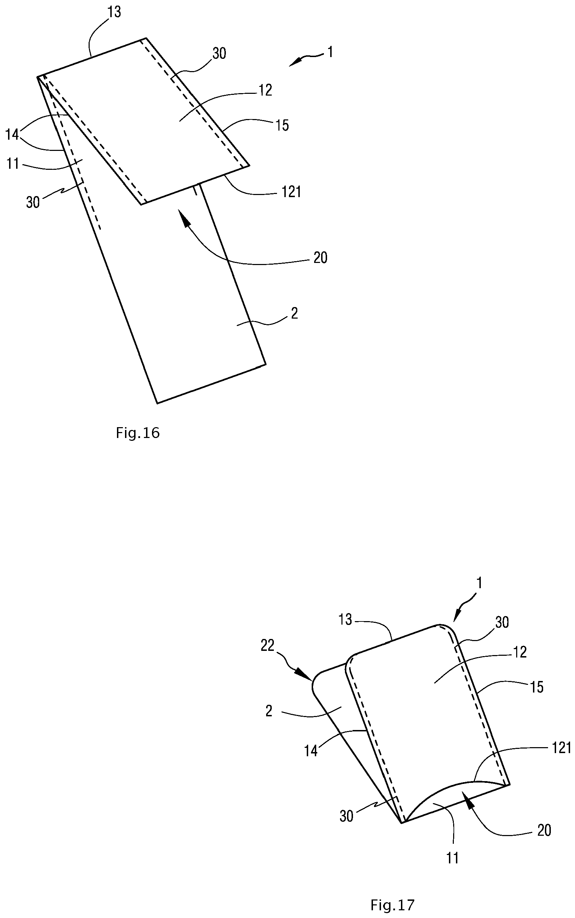

For example, Fig. 16 shows a schematic diagram of the processing of the cleaning sleeve for cleaning the oral cavity according to another embodiment of the present invention. It can be seem from Fig. 16 that the cleaning sleeve of the present embodiment can be completely formed by folding and cutting a single piece of fabric (under the circumstance that the grip portion 2 is integrally extended from the working layer 11). In other words, the working layer 11 and the sleeve layer 12 of the cleaning sleeve of the present embodiment are formed by the same piece of folded fabric (in this case, the grip portion 2 and the working layer 11 can be formed by the same piece of fabric, or by connecting two separate structures). The working layer 11 and the sleeve layer 12 form a seamless head 13 at the top of the cleaning sleeve. The working layer 11 and the sleeve layer 12 form a first edge 14 and a second edge 15 with bonded seams 30 on both sides of the cleaning sleeve. The first edge 14 and second edge 15 are formed by laminating the working layer 11 and the sleeve layer 12 and then are ultrasonically bonded to form the bonded seam 30 (as shown in the dotted line).

The sleeve portion 1 of the cleaning sleeve shown in Fig. 16 which is not part of the present invention, is a double-layer structure consisting of the working layer 11 and the sleeve layer 12. The common compositing method is to place two separate pieces of nonwoven fabrics up and down, then ultrasonically bond the nonwoven fabrics and then die cut the nonwoven fabrics. The cleaning sleeve of the present embodiment may be processed by a method that a piece of nonwoven fabric is folded to form the two-layer structure of the sleeve portion 1 so that the step of forming two separate fabric layers is eliminated and one nonwoven fabric layer can be simply folded to form two fabric layers. Meanwhile, waste of materials caused by die cutting of a head bonding part is also avoided (in the present embodiment, the head has no seam so that it is unnecessary to apply die cutting to cut off excess parts and the waste of materials can be avoided). Thus, the process is simple and reliable; and the productivity and production efficiency can be greatly improved. In addition, the method of folding in the middle is adopted so that the head 13 of the cleaning sleeve can be formed without ultrasonic bonding, thereby reducing processing losses. The top also smartly avoids the rough corners caused by ultrasonic bonding technology, thereby reducing the discomfort to the oral cavity caused by friction and offering better user experience.

Fig. 17 is a schematic diagram of the cleaning sleeve for cleaning the oral cavity according to another embodiment of the present invention in a packaged state. As shown in the figure, the grip portion 2 can be folded to cover the working layer 11 to protect the surface in the packaged state of the cleaning sleeve of the present invention. Since the working layer 11 of the cleaning sleeve needs to be in contact with the oral cavity, the surface should be protected during transport and storage to avoid contamination with bacteria. A common way is to use a separate piece of protective film or isolation paper for protection, which will require extra cost and reduce production efficiency. In the present embodiment, the grip portion 2 is directly folded to cover and protect the working layer 11 in the packaging process after production, which can omit the use of the protective film or isolation paper so that the safeness and hygiene of the product is greatly improved. After a user purchases the cleaning sleeve, the grip portion 2 can be unfolded from the state of covering the working layer 11 to fix and prevent the cleaning sleeve from slipping from the finger or tool while in use. Moreover, in the unfolding process, the working layer 11 does not come into contact with the finger or other outside parts, thereby reducing the probability of being contaminated by bacteria.

In the product setting of the present embodiment, the grip portion 2 does not only have the feature to fix the product in place, but can be folded to cover and protect the surface of the working layer 11. A structure with two different functions has never been displayed in the prior art, but is apparent to those skilled in the art, and is an outstanding feature of creativity of the present invention.

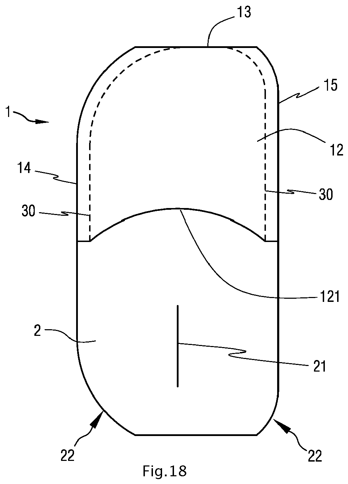

Furthermore, the two corners at the end of the grip portion 2 form the end arc angles 22. Based on the above embodiment in which the head 13 of the cleaning has two arc corners at two corners, if the two corners at the end of the grip portion 2 have the same end arc angle as the arc angle at the corresponding side of the head 13 of the cleaning sleeve, the end arc angles 22 can overlap with the arc angles at both sides of the head 13 when folding the grip portion 2 to cover the working layer 11 (Fig. 18 shows a structural schematic diagram of another cleaning sleeve of the present application). No protruded part is formed during packaging, thereby avoiding affecting normal use of the user due to wrinkle parts formed at the protruded parts. Therefore, the user may have a better visual experience and further improved use experience.

## Claims

1. A cleaning sleeve for cleaning the oral cavity, suitable to be applied over a finger or a tool to clean the inside of the oral cavity, wherein that the cleaning sleeve is a flat-layered structure comprising a sleeve portion (1) formed by laminated sheet-like fabrics and a grip portion (2) formed by a single-layer sheet-like fabric;
the sleeve portion (1) is applied over the finger or the tool; the grip portion (2) is held in the palm while in use;
the sleeve portion (1) formed by the laminated sheet-like fabrics comprises a working layer (11) and a sleeve layer (12); the working layer (11) and the sleeve layer (12) form a head (13) at the top of the cleaning sleeve;
two sides of the cleaning sleeve are provided with a first edge (14) and a second edge (15);
the working layer (11) and the sleeve layer (12) are respectively connected to the head (13), the first edge (14) and the second edge (15) to form a sleeve-like structure with an inserting hole (20);
the grip portion (2) is integrally extended from the working layer (11);
the grip portion (2) can be folded to cover and protect the surface of the working layer (11) in a packaged state; and while in use, the grip portion (2) can be unfolded from a state of covering the working layer (11) to fix the cleaning sleeve in place and prevent the cleaning sleeve from slipping from the finger or tool;
**characterized in that**
an opening (21) is formed on the grip portion (2) to help fix the cleaning sleeve in place by inserting the thumb into the opening (21).

2. The cleaning sleeve according to claim 1, wherein the sleeve layer (12) has an inserting edge (121) opposite to the head (13) of the cleaning sleeve; and the inserting edge (121) has an inserting structure for facilitating insertion; the inserting structure is a curved edge of the inserting edge (121) facing or deviating from the head (13).

3. The cleaning sleeve according to claim 1, wherein arc angles are formed on two corners of the head (13) of the cleaning sleeve; and the arc angle of the head (13) of the cleaning sleeve at an operation side is greater than the other one.

4. The cleaning sleeve according to claim 3, wherein both corners at the end of the grip portion (2) are provided with the same end arc angles (22) as the arc angles at the corresponding sides of the head (13) of the cleaning sleeve.

5. The cleaning sleeve according to claim 1, wherein the working layer (1) and the sleeve layer (12) are folded by the same piece of fabric.

6. The cleaning sleeve according to claim 1, wherein the cleaning sleeve is formed by ultrasonically bonding degradable nonwoven fabric; and the degradable nonwoven fabric is formed by blended polyactic acid (PLA) fiber and viscose fiber.

## Patentansprüche

1. Reinigungshülse zur Reinigung einer Mundhöhle, die geeignet ist, über einem Finger oder einem Werkzeug gestülpt zu werden, um das Innere der Mundhöhle zu reinigen, wobei die Reinigungshülse eine flache Schichtstruktur ist, die einen Hülsenabschnitt (1), der aus laminierten blattartigen Stoffen gebildet ist, und einen Griffabschnitt (2) umfasst, der aus einem einlagigen blattartigen Stoff gebildet ist;
wobei der Hülsenabschnitt (1) über dem Finger oder dem Werkzeug gestülpt wird und der Griffabschnitt (2) bei der Benutzung in der Handfläche gehalten wird;
wobei der durch die laminierten blattartigen Stoffen gebildete Hülsenabschnitt (1) eine Arbeitsschicht (11) und eine Hülsenschicht (12) umfasst, wobei die Arbeitsschicht (11) und die Hülsenschicht (12) einen Kopf (13) an der Oberseite der Reinigungshülse bilden;
wobei zwei Seiten der Reinigungshülse mit einer ersten Kante (14) und einer zweiten Kante (15) versehen sind;
und wobei die Arbeitsschicht (11) und die Hülsenschicht (12) jeweils mit dem Kopf (13), dem ersten Rand (14) und dem zweiten Rand (15) verbunden sind, um eine hülsenartige Struktur mit einem Einsteckloch (20) zu bilden;
wobei sich der Griffabschnitt (2) einstückig von der Arbeitsschicht (11) erstreckt;
wobei der Griffabschnitt (2) gefaltet werden kann , um die Oberfläche der Arbeitsschicht (11) in einem verpackten Zustand zu bedecken und zu schützen; und wobei während der Verwendung der Griffabschnitt (2) aus einem die Arbeitsschicht (11) bedeckenden Zustand entfaltet werden kann, um die Reinigungshülse an Ort und Stelle zu fixieren und zu verhindern, dass die Reinigungshülse aus dem Finger oder dem Werkzeug abrutscht;
**dadurch gekennzeichnet, dass** am Griffabschnitt (2) eine Öffnung (21) ausgebildet ist, um die Fixierung der Reinigungshülse durch Einführen des Daumens in die Öffnung (21) zu unterstützen.

2. Reinigungshülse nach Anspruch 1, wobei die Hülsenschicht (12) eine dem Kopf (13) der Reinigungshülse gegenüberliegende Einführkante (121) aufweist; wobei die Einführkante (121) eine Einführstruktur zur Erleichterung der Einführung aufweist; wobei die Einführstruktur eine gekrümmte Kante der Einführkante (121) ist, die dem Kopf (13) zugewandt ist oder von diesem abweicht.

3. Reinigungshülse nach Anspruch 1, wobei an zwei Ecken des Kopfes (13) der Reinigungshülse Bogenwinkel ausgebildet sind, wobei der Bogenwinkel des Kopfes (13) der Reinigungshülse an einer Arbeitsseite größer als der andere ist.

4. Reinigungshülse nach Anspruch 3, wobei die beiden Ecken am Ende des Griffabschnitts (2) mit den gleichen Endbogenwinkeln (22) wie die Bogenwinkel an den entsprechenden Seiten des Kopfes (13) der Reinigungshülse versehen sind.

5. Reinigungshülse nach Anspruch 1, wobei die Arbeitsschicht (1) und die Hülsenschicht (12) aus demselben Stoffstück gefaltet sind.

6. Reinigungshülse nach Anspruch 1, wobei die Reinigungshülse durch Ultraschallverfestigung von abbaubarem Vliesstoff gebildet wird und der abbaubare Vliesstoff aus einer Mischung aus Milchsäurefasern (PLA) und Viskosefasern besteht.

## Revendications

1. Manchon de nettoyage pour nettoyer la cavité buccale, applicable sur un doigt ou un outil pour nettoyer l'intérieur de la cavité buccale, dans lequel le manchon de nettoyage a une structure en couche plate comprenant une portion de manchon (1) formée par des tissus stratifiés en forme de feuille et une portion de préhension (2) formée par un tissu de monocouche en forme de feuille ;
la portion de manchon (1) est appliquée sur le doigt ou l'outil ; la portion de préhension (2) est maintenue dans la paume lors de l'utilisation ;
la portion de manchon (1) formée par les tissus stratifiés en forme de feuille comprend une couche de travail (11) et une couche de manchon (12) ; la couche de travail (11) et la couche de manchon (12) forment une tête (13) à la partie supérieure du manchon de nettoyage ;
le manchon de nettoyage est pourvu sur ses deux côtés d'un premier bord (14) et d'un deuxième bord (15) ;
la couche de travail (11) et la couche de manchon (12) sont respectivement connectées à la tête (13), le premier bord (14) et le deuxième bord (15), de sorte de former une structure en forme de manchon avec un trou d'insertion (20) ;
la portion de préhension (2) s'étend intégralement à partir de la couche de travail (11) ;
la portion de préhension (2) est pliable pour couvrir et protéger la surface de la couche de travail (11) à l'état emballé ; et lors de l'utilisation, la portion de préhension (2) est dépliable à partir d'un état de recouvrement de la couche de travail (11), de sort de maintenir le manchon de nettoyage en place et empêcher le manchon de nettoyage de glisser sur doigt ou l'outil ;
**caractérisé en ce que**,
une ouverture (21) est formée sur la portion de préhension (2) pour aider à maintenir le manchon de nettoyage en place en insérant le pouce dans l'ouverture (21).

2. Manchon de nettoyage selon la revendication 1, dans lequel la couche de manchon (12) présente un bord d'insertion (121) à l'opposé de la tête (13) du manchon de nettoyage ; et le bord d'insertion (121) a une structure d'insertion pour faciliter l'insertion ; la structure d'insertion étant un bord incurvé du bord d'insertion (121) faisant face à la tête ou s'écartant de celle-ci (13).

3. Manchon de nettoyage selon la revendication 1, dans lequel des angles d'arc sont formés à deux coins de la tête (13) du manchon de nettoyage ; et l'angle d'arc de la tête (13) du manchon de nettoyage sur le côté d'opération est supérieur à l'autre.

4. Manchon de nettoyage selon la revendication 3, dans lequel les deux coins à l'extrémité de la portion de préhension (2) présentent des mêmes angles d'arc d'extrémité (22) que les angles d'arc sur les côtés correspondants de la tête (13) du manchon de nettoyage.

5. Manchon de nettoyage selon la revendication 1, dans lequel la couche de travail (1) et la couche de manchon (12) sont pliables par une même pièce de tissu.

6. Manchon de nettoyage selon la revendication 1, dans lequel le manchon de nettoyage est formé par collage à ultrason d'un tissu non tissé dégradable ;
et le tissu non tissé dégradable est formé par des fibres d'acide polyactique (PLA) mélangées et d'une fibre de viscose.
